# EUROPEAN PATENT SPECIFICATION

(11) **EP 2 149 374 B1**
(45) Date of publication and mention of the grant of the patent: **10.10.2012**
(21) Application number: 08766681.4
(22) Date of filing: 28.05.2008
(51) Int. Cl.: A61K 31/496, A61K 31/375, A61P 31/00, A61P 13/00

(54) **PHARMACEUTICAL COMPOSITION COMPRISING THE COMBINATON OF THE ANTI-MICROBIAL AGENT CIPROFLOXACIN AND THE ANTIOXIDANT AGENT ASCORBIC ACID FOR THE TREATMENT OF URINARY INFECTIONS**
PHARMAZEUTISCHE ZUSAMMENSETZUNG AUS DEM ANTIMIKROBIELLEN WIRKSTOFF CIPROFLOXACIN UND DEM ANTIOXIDANTIUM ASCORBINSÄURE ZUR BEHANDLUNG VON HARNWEGSINFEKTIONEN
COMPOSITION PHARMACEUTIQUE COMPRENANT UNE COMBINAISON FORMÉE PAR L'AGENT ANTIMICROBIEN CIPROFLOXACINE ET L'AGENT ANTIOXYDANT ACIDE ASCORBIQUE POUR LE TRAITEMENT DES INFECTIONS URINAIRES

(30) Priority: 28.05.2007 MX MX07006334
(43) Date of publication of application: 03.02.2010
(73) Proprietor: WORLD-TRADE IMPORT-EXPORT, WTIE, A.G., 6320 Zug (CH)
(72) Inventor: GARCÍA ARMENTA, María Elena, C.P. 45020 Guadalajara, Jalisco (MX); SANTOS MURILLO, Josefina, C.P. 44600 Zapopan, Jalisco (MX); ÁLVAREZ OCHOA, Víctor Guillermo, C.P. 45222, Zapopan, Jalisco (MX)
(74) Representative: Carvajal y Urquijo, Isabel
(86) International application number: PCT/MX2008/000066
(87) International publication number: WO 2008/147167

(56) References cited:
- EP-A2- 1 880 719
- WO-A1-2004/089418
- WO-A2-02/089744
- WO-A2-2008/060865
- US-A1- 2005 239 722
- HAGER J ET AL: "Malakoplakia of the colon in an 8-year-old boy" PADIATRISCHE PRAXIS 200603 DE, vol. 68, no. 1, March 2006 (2006-03), pages 91-98, XP009140396 ISSN: 0030-9346
- MOTOYA TOSHIRO ET AL: "Effects of ascorbic acid on interactions between ciprofloxacin and ferrous sulphate, sodium ferrous citrate or ferric pyrophosphate, in mice" JOURNAL OF PHARMACY AND PHARMACOLOGY, vol. 52, no. 4, April 2000 (2000-04), pages 397-401, XP002606727 ISSN: 0022-3573
- GOSWAMI M. ET AL.: 'Involvement of reactive oxygen species in the action of Ciprofloxacin against Escherichia coli' ANTIMICROBIAL AGENTS AND CHEMOTHERAPY vol. 50, no. 3, March 2006, pages 949 - 954, XP008125723

## Description

### FIELD OF THE INVENTION

The present invention is related to the pharmaceutical industry and concerns a pharmaceutical composition comprising the synergic combination of an anti-microbial agent, such as: Ciprofloxacin and an antioxidant agent, such as: Ascorbic Acid (Vitamin C), as well as pharmaceutically acceptable excipients; which are formulated in a single dosage unit for oral administration, intended for the treatment of Urinary Infections.

The combination of these active principles produce a major synergic effect when administered in combination in a single dosage unit, as opposed to when these are independently administered de forma, providing benefits such as: reduced concentrations of the active principles contained in the formula, reduced administered doses, optimization of the therapeutic effect and decreased risk of adverse events.

### BACKGROUND OF THE INVENTION

The urinary system is composed of the kidneys, ureters, bladder and urethra. The kidneys filter and extract all the residues and water from circulating blood in order to produce urine, generating about 1 and 1.5 1 of urine/day in an adult (this value is lower in children depending on the age). Urine is conveyed from the kidneys (where it is formed) through 2 narrow tubes called: ureters, these tubes terminate in the bladder, an organ where urine is stored until it is expelled from the body by the urethra, another tube located in the lower part of the bladder and which terminates at the end of the penis in male subjects and in the frontal part of the vagina in female subjects.

Urinary infections (UI) are one of the most frequent diseases. The term urinary infection refers to any infection caused by a pathogenic agent (bacteria in most cases), which affects any of the segments comprising the urinary system: Kidneys, ureters, bladder or urethra. The manifestation of a urinary infection implies the existence of microorganisms in urine at high titers; however, this is not exclusive, as in some cases, these levels are low.

In its normal state, urine does not contain bacteria. Normally, bacteria can be found all over the skin and can be present in the perineal (anal) or genital region and move towards the urethra to the bladder. When this happens, bacteria infect and inflame the bladder, causing pain in the lower abdominal region and a burning sensation during urination, resulting in the manifestation of an infection called: Cystitis. In female subjects, the urethral orifice is located a few centimeters away from the anus, whereby, bacteria can easily enter the urinary system and cause said infection.

Urine is sterile, which means that, normally, it does not contain pathogenic agents. Usually, urinary infections are caused by microorganisms located in the large intestine and the most frequent microorganism is called Escherichia coli. If the bladder infection (cystitis) is not treated promptly, it may ascend through the ureter to the kidney and produce an infection called: Pyelonephritis, which is a kidney infection manifested by back pain, fever, and if the infection is not treated promptly, bacteria can reach the blood flow and cause a very severe infection which can cause death, called: sepsis.

The infections above are the most frequent diseases suffered by the patients who go to medical consultation; however, there are other types of infections manifested in the urinary system, including: Ureteritis: Is an infection in one ureter and generally, it is an extension of a kidney or bladder infection; Urethritis: Which is an infection in the urethra, among others. Pyelonephritis is also known as Higher Urinary Tract Infection and Cystitis and Urethritis are known as Lower Urinary Tract Infections.

Any abnormality in the urinary apparatus blocking the urine flow may produce urinary infection. Some examples of factors causing urinary infections are the following: in female subjects, the length of the feminine urethra is short allowing bacteria to reach the perineal region, to the initial part of the urinary apparatus; in male subjects, an enlarged prostate which tends to retain urine, due to the fact that an enlarged prostate partially or totally blocks the segment corresponding to the urethra; people with low liquid intake may develop the formation of kidney stones; while a probe may be placed with extra caution, the presence of probes or catheters, an infection may occur a few days after it has been placed; also, diabetic people have a major urinary infection risk, due to their susceptibility to any type of infection; the same occurs in geriatric or weakened patients, as their immune defenses are very low; people who voluntarily retain urine, as it is important to urinate as soon as the urge arises; the use of tight clothes, as these exert too much pressure and cause the urine to reflow towards the inner part of urinary ducts, promoting the contamination in this area. Urine infections are less frequent in children and young people. Women using diaphragms are more susceptible to develop more infections that those using other contraceptive methods. Most people suffering from a urinary infection have symptoms; however, in some of these cases, symptoms are not observed. The most frequent symptoms are: Urge to urinate, frequent urination in lower amounts than usual and remain with the urge to urinate (pollakiuria), burning sensation during urination (dysuria), pain or pressure in the low abdomen at the end of the emission, cloudy or pink urine, fever, tiredness, decline, vomit, nausea or back pain depending on the localization of the infection.

Most of the microorganisms causing these infectious processes are gram negative bacilli, which have a great genetic plasticity to express and obtain decisive resistance levels against anti-microbial agents.

Generally, urinary infections are mono-bacterial infections. Bacteria causing one of the most frequent UI are the following: Escherichia coli (85%), followed by Proteus mirabilis, Klebsiella pneumoniae and Streptococcus agalactiae (in pregnant female subjects, geriatric and diabetic patients). With less frequency, it is caused other enterobacteriaceae, such as: Pseudomonas aeruginosa, Enterococcus spp. or non-bacterial microbes such as Chlamydia and Mycoplasma species. Staphyloccocus saprophyticus is a relatively frequent agent in lower UI, which is typically found in sexually active female subjects. Staphylococcus aureus can be found in patients with vesical probes or haematogenic UIs. Staphylococcus epidermidis is considered a cutaneous polluting agent and it rarely causes UIs.

Statistically, urine infections are rather frequent, as they are surpassed only by respiratory infections. Women are affected more often than male subjects, and one in 5 female subjects will have at least one urinary infection at some point of her life.

Other important reasons for the rational use of antibiotics in urinary infection antibiotic plans are the recurrence of lower infections in young female subjects, as well as the selection of safe antibiotics in pregnant patients.

The diagnosis of a UI is carried out considering the following: The predisposing factors of the patients and the symptoms they describe and the physical examination realized; however, the fist and most important exam which must be ordered by the physician is a urinalysis to confirm the presence of red blood cells, white blood cells and bacteria. It is convenient to carry out a urine culture to know the type of bacteria present and, thus, be able to administer a suitable treatment. The physician is likely order a simple radiography to confirm the presence of kidney stones and/or a kidney ecography to diagnose duct dilatation (hydronephrosis). The commonly administered treatment consists of antibiotics for 4 to 5 days in the case of a simple infection and de 2 to 3 weeks in case of a kidney (acute pyelonephritis) or prostate (acute prostatitis) infection. Though urine cultures, it is possible to identify the microorganism causing the infection and the antibiogram will reveal the most appropriate antibiotic for its treatment. Nowadays, urinary infections are a very frequent cause of medical consultation in primary care. In many cases, this makes it necessary to start an empirical antibiotic treatment, as the cause and severity of the infection are commonly unknown, until results of microbiological studies are obtained.

At present, most of the commercially available drugs for the treatment of Urinary Infections are composed of active principles independently formulated, which perform such as therapeutic activity that, in most cases, it is limited with respect of the microorganism diversity present, which cause a urinary infection.

WO 02/089744 A2 discloses combined treatments and methods of treatment of *Mycoplasma* and *Mycoplasma*-like organism infections with antibiotics combined with antioxidants to reduce oxidative stress.

HAGER J ET AL: "Malakoplakia of the colon in an 8-year-old boy", PADIATRISCHE PRAXIS 200603 DE, vol. 68, no. 1, March 2006 (2006-03), pages 91-98, ISSN: 0030-9346 reports one case of Malakoplakia treated with ciprofloxacin and pyridostigmine bromide, wherein vitamin C is co-administered as coadyuvant of the cholinergic drug.

### SUMMARY OF THE INVENTION

In order to provide a pharmaceutical alternative to eliminate the presence of pathogenic agents found in urine and further stop bacteria growth progression and, thus, preventing the apparition of a complex urinary infection, the present invention was developed describing a pharmaceutical composition comprised of the combination of an anti-microbial agent and an antioxidant agent, which act in a synergic manner and are formulated in a single dosage unit for oral administration, providing benefits such as: reduced concentrations of the active principles contained in the formula, reduced administered doses, optimization of the therapeutic effect and decreased risk of adverse events.

### DETAILED DESCRIPTION OF THE INVENTION

**Antimicrobial agents** are defined as natural (fungi or bacteria), synthetic or semi-synthetic chemical substances having the ability to inhibit the development or cause the destruction of pathogenic microorganisms, which may produce an infection. These are systemic agents which reduce and control the presence of pollutant microorganisms in the host.

Their toxic efficacy results from its ability to inhibit a specific and essential biochemical reaction for bacteria development. In order to exert its action, an antibiotic must contact the infected site, break through the bacteria (by means of diffusion or active transport) and reach the required concentration at an intracellular level.

Once within cells, antibiotics are able to exert two types of effects: Bacteriostatic effect, which prevents the development of microorganisms without causing their destruction, thus, these are capable to multiply again once the effect has passed; or Bactericide effect, which produces the destruction of microorganisms (lethal effect).

Antibiotics are able to exert their action in one of the following structures or functions: 1.- Inhibition of cell wall synthesis (Penicillins, aminopenicillins, carboxypenicillins, cephalosporins, B-lactams, carbapenems); 2.- Alteration in cytoplasmic membrane or cell permeability (Polymixins, imidazoles and polyenes); 3.- Inhibition of protein synthesis (Tetracyclines, macrolides, aminoglycosides, amphenicols, lincosamides) and 4. - Blocking or inhibition of nucleic acid synthesis (Sulphonamides, quinolones, diaminopyrimidines, ansamicines). Quinilones are part of an antibiotic family known since the 60's. Nalidixic acid was the fist quinolone used, which in combination with pipemidic acid, constituted the firs generation of quinolones. The later has a wider action spectrum and better pharmacokinetic properties. Both were considered urinary antiseptics. Since then, a great number of quinolones have been synthesized and researched, in order to increase their activity and action spectrum, as well as to reduce their adverse effects. Second-generation quinolones are fluorinated derivatives or fluoroquinolones (FQ). There is a third generation composed of bi- and trifluorinated derivatives and the fourth generation is currently under development.

The first FQ introduced was norfloxacin, which constituted a major progress due to its greater power and wider antibacterial spectrum. Subsequently, the following FQ were introduced: Ciprofloxacin, Ofloxacin, Enoxacin, Lomefloxacin, Temafloxacin, Levofloxacin, Sparfloxacin, Trovafloxacin, Grepafloxacin, Gatifloxacin, Moxifloxacin and Gemifloxacin. Some of these were withdrawn from the market after obtaining commercial approval, or their use has been restricted due to their toxic effects (Sparfloxacin, Trovaloxacin, Grepafloxacin).

The first quinolones acted only against gram-negative aerobic bacteria and were effective for the treatment of gastrointestinal and urinary infections. The introduction of a fluorine atom in the basic molecule of quinolones produced fluoroquinolones (FQ), which have a stronger power, wider anti-bacterial spectrum, longer half-life and, with the exception of norfloxacin, they reach good serum levels, thus, it is possible to treat systemic infections.

FQs are bactericide agents which inhibit DNA girase, an enzyme involved in double-spiral-DNA folding, which is vital for the three-dimensional structure of genetic material, exerting its action at intracellular level.

Basically, its activity depends on two factors, such as: The ability of breaking through the cytoplasmatic barrier and the affinity for bacteria DNA-girases, enabling them to reach tissue concentrations equal or greater than those obtained in serum; reaching high concentrations in the urinary tract (urine, kidney, prostate tissue) and pulmonary tissue.

**Ciprofloxacin** has a wide "in vitro" activity spectrum and a good activity when compared to gram-negative bacteria. It is active against Chlamydia, Mycoplasma and some other micobacteria; presenting further activity against Pseudomona aeruginosa and, in a very specific manner, against Staphylococcus. Absorption of Ciprofloxacin orally administered is 95% in two hours and 100% in three hours; it provides a bioavailability from 70% to 80% and maximum haematic concentrations are reached about one hour after its administration. Ciprofloxacin provides a high distribution level and reaches higher concentrations when com to those found in serum in several tissues and liquids. Regardless of the dose, its half-life was 4 hours and it bound to plasmatic proteins in 30%.

Ciprofloxacin is mainly eliminated by renal excretion, through glomerular filtration and tubular excretion as Ciprofloxacin without change and in the form of its four active metabolites: oxyciprofloxacin, sulphociprofloxacin, desmethylciprofloxacin and formylciprofloxacin. As alternative elimination path, it has the hepatobiliar system. Ciprofloxacin can be administered either orally or parenterally, which allows the application of a sequential therapy from intravenous to oral administration.

Evolution has resulted in cells having protection mechanisms against the noxious effects of free radicals based on a complex defense mechanism constituted by antioxidant agents.

**Antioxidants** are a heterogeneous set of substances comprised of vitamins, minerals, natural pigments and other vegetal compounds and enzymes blocking the noxious effect of free radicals. The term "antioxidant" refers to agents preventing the detrimental oxidation of other chemical substances, which are substances naturally found in the organism, involved in several metabolic processes or those produced by exogenous factors, such as ionizing radiations. A free radical is a (organic or inorganic) molecule, containing unpaired electrons in orbitals involved in chemical binding; generally, it is extremely unstable, therefore, its reactive power is high. These can be formed by electron loss or gain; in the first case, it is an oxidation process and in the second one, a reduction process. These can be synthesized in a laboratory, formed in the atmosphere by means of radiation or formed in living organisms (including the human body) by oxygen contact. These alter cell membranes and attack cell genetic material, especially DNA.

Cell defense mechanisms against free radicals can be enzymatic and are called: **Endogenous antioxidants,** including the enzymes superoxidodismutase, catalase, glutathione peroxidase, glutathione and the coenzyme Q; or **exogenous antioxidants,** which enter the organism through food. When said antioxidants reach the cells, they settle in their membranes and protect them against lipoperoxidation, as in the case of vitamin C, E and β-carotene.

Deoxyribonucleic acid (DNA), main chromosome component carrying genetic information of cells, also constitutes one of the major targets of free radicals. The mutations resulting from the damage produced by the latter to DNA could ultimately lead to an uncontrolled loss of cell division and, thus, the formation of tumors.

There are particular situations which increase free radical production, including: Intense physical exercise, stress, environmental pollution, smoking, alcoholism, unsuitable diets and over exposition to solar radiation.

In recent years, the role of antioxidants in maximum impact diseases, such as cardiovascular diseases, several types of cancer, aids and even other directly related to the aging process, such as cataracts, Alzheimer's disease and other nervous system alterations have been studied.

**Ascorbic Acid or Vitamin C** is classified, along with vitamins B, in the hydrosoluble vitamin group, as it is involved in keeping strong bones, gums, teeth and blood vessels, and it is very effective in the formation and keeping of collagen (acting as cofactor in the hydroxylation of the monoacids lysine and proline). Vitamin C protects vitamin A and vitamin E against oxidation as well some compounds pertaining to B-complex (thiamine, riboflavin, folic acid and pantothenic acid). Ascorbic Acid develops anti-infectious and antitoxic actions. It aids in the absorption of non-haem iron in the organism and metabolism of fats.

Vitamin C is necessary for the growth and regeneration of connective tissue in every area of the body. It is necessary to form collagen, an important protein used in the formation of the skin (softer skin, due to the binding of cells needing this vitamin to achieve said process), cicatricial tissue, tendons, ligaments and' blood vessels. Vitamin C is essential for the cicatrisation of wounds and the regenerations and keeping of cartilage, bones and teeth.

Vitamin C is one of several antioxidants, similar to other two well known antioxidants such as: vitamin E and beta-carotene. Antioxidants are nutrients blocking part of the damage caused by free radicals, which are by-products resulting from the transformation of food into energy carried out by the body.

Vitamin C has several healing characteristics, such as the so-called treatment for minor respiratory diseases (cold, flu); and it is also used for: preventing premature aging, facilitating the absorption of other vitamins and minerals, as antioxidant agent, preventing degenerative diseases, such as: atherosclerosis, cancer, Alzheimer's disease, preventing heart and urinary diseases, and strengthening the immune system. A human being's minimum vitamin C requirements are < 60 mg/day.

There are a number of pharmaceutical products commercially available pretended for the treatment of urinary infections, containing several active principles independently formulated, causing an inferior therapeutic effect and the necessity to administer greater doses of said products, resulting into an increase in the number of patients withdrawing or dropping out from the treatment.

The pharmaceutical composition object of the present invention is comprised of the synergic combination of an anti-microbial agent, such the active principle: Ciprofloxacin and an antioxidant agent, such as the active principle: Ascorbic Acid (Vitamin C), as well as pharmaceutically acceptable excipients, which are formulated in a single dosage unit for oral administration, is indicated for the treatment of Urinary Infections, and has been developed considering the fact that both active principles have great efficacy and capacity to attack and eliminate the presence of several pathogenic agents found in urine, as well as to stop the bacterial growth progression, thus preventing the apparition of a complex or severe urinary infection, as well as providing an increased therapeutic effect, an increase in the treatment adherence and a reduction in the infection in a shorter period.

Dosage of Vitamin C and Ciprofloxacin is determined considering several factors, including: Severity of the infection, sensibility of the organisms causing the infection, age, weight and patient renal function quality.

In order to assess the efficiency and tolerance of the pharmaceutical composition object of the present invention, as well as the synergetic effect of the active principles, Ciprofloxacin and Ascorbic Acid, combined in a single dosage unit, a comparative clinical study was conducted, wherein the above active principles were individually administered, as well as the combination thereof.

### COMPARATIVE STUDY OF CIPROFLOXACIN, VITAMIN C AND THE COMBINATION OF CIPROFLOXACIN AND VITAMIN C IN HEALTHY SUBJECTS

Urinary tract infection constitutes the most frequent urologic disease and is one of the most common infections in human beings. Urinary infection encompasses several clinical conditions, so the proper study thereof is very important for a suitable diagnose and treatment, in view of the extensive and not inconsiderable hospital and healthcare expenses these involve. A comparative clinical study was conducted in 150 patients with a non-complex acute urinary infection diagnose, wherein the' effect of Ciprofloxacin, Vitamin C and the combination of both active principles was evaluated.

### Materials and Methods.

Inclusion criteria were the following:
- Patients from 18 to 75 years of age.
- Patients having a non-complex acute urinary infection diagnose.
> 12 leukocytes per mm3 in urine sediments.
- Urine counts of > 100,000 UFC/ml.
- Urine pH over 6.0.
- Microorganism identification.

Exclusion criteria were the following:
- Patients suffering from concomitant diseases (diabetes mellitus, arterial hypertension, prostate hyperplasia).
- Patients using other drugs. - Patients having a chronic and complex urinary infection diagnose.
- Patients having urocultives with a non-Ciprofloxacin-sensitive microorganism.

150 patients enrolled in the study and their average age was 37, including 120 female subjects and 30 male subjects (Table 1).

The 150 patients were randomized in 3 groups (50 per group), and were subject to a general urine examination and urocultive by antibiogram (in order to identify the causal agent and its sensitivity to Ciprofloxacin); group 1 received Ciprofloxacin 500 mg. Only twice a day for 5 days,- group 2, received Vitamin C 100 mg. Twice a day for 5 days; group 3 received the combination of Ciprofloxacin 500 mg. / Vitamin C 100 mg. twice a day for 5 days.

### Results.

The 150 enrolled patients finished the study, Table 1 shows patient demographic data, the average age was 37 and the patients included were 120 female subjects and 30 male subjects.

**Table 1. Demographic Data from Patients suffering from non-complex acute urinary infections.**

| No. Patients | Average Age | Sex |
|---|---|---|
| N=150 | 37 years | old 120 (m) 30 (h) |

General urine examination determined the color, appearance and density thereof, as well as the pH, Proteins, Glucose, Ketones, Blood, Bilirubin, Urobilinogen, Nitrites, Density and Leukocytes. From the results, relevant data corresponding to the baseline and up to Day 5 of the Treatment is appended herein (Table 2 and 3).

**Table 2. Leukocytes per mm³ in baseline urine sediments and up to Day 5 of the treatment.**

| Group | No. Patients | Leukocytes (Day 1) | Day 5 |
|---|---|---|---|
| 1 (Cipro) | n=50 | > 16 | < 5 |
| 2 (Vit C) | n=50 | > 13 | < 10 |
| 3 (Comb.) | n=50 | > 17 | < 3 |

**Table 3. Baseline urine pH and up to Day 5 of the treatment.**

| Group | No. Patients | pH (Day 1) | pH (Day 5) |
|---|---|---|---|
| 1 (Cipro) | n=50 | 7.5 | 6.0 |
| 2 (Vit C) | n=50 | 7.6 | 5.0 |
| 3 (Comb.) | n=50 | 7.5 | 3.0 |

The urocultive by antibiogram determined the causal agent (E. coli) and the sensitivity thereof to the Ciprofloxacin treatment, relevant data is appended herein (Table 4).

**Table 4. Urine Counts in UFC/ml. (Colony-Forming Units).**

| Group | No. Patients | UFC/ml (Day 1) | Day 5 |
|---|---|---|---|
| 1 (Cipro) | n=50 | >100,000/ml. | 15/ml. |
| 2 (Vit C) 90.000/ml. | n=50 | >100,000/ml. | |
| 3 (Comb.) | n=50 | >100,000/ml. | 2/ml. |

### Conclusions

As can be noted, a diagnose of non-complex acute urinary infection was made, laboratory examination data show the severity of the process in patients in Day 1 (baseline) and the improvement with the treatment in Day 5 can be observed; however, the improvement is evident with the combined treatment (P<0.05). This improvement is due to the synergic effect shown by both components Ciprofloxacin /Vitamin C, as, while it is true that Ciprofloxacin, a wide spectrum fluoroquinolone, has a great activity against Escherichia coli, as was observed in the Ciprofloxacin-treated group 1; the combination of Ciprofloxacin / Vitamin C was found to improve even further the healing process in group-3 patients.

It is worth to mention that when administering the combination, there was a change in the urine pH reflecting an improved Ciprofloxacin activity, in turn and by itself, as, an antioxidant agent, Vitamin C has a great influence over non-bacterial progression.

Vitamin C activity to improve urine pH in these patients is not sufficient by itself to achieve a healing process in patients suffering from acute and non-complex urinary tract infections. The object of the present invention is to provide a pharmaceutical alternative for patients suffering from urinary infections, comprising the combination of Ciprofloxacin and Vitamin C, which produces a synergic effect generating an effective therapeutical activity at different levels, thus causing a decrease in the number of administered doses, reducing the risk of adverse events and increasing the treatment adherence by patients, which, in some cases, is extended up to 14 days.

### NOVELTY OF THE INVENTION

Having described the present invention, it is considered as novelty, therefore, it claims the property of the contents stated in the appended claims.

## Claims

1. A pharmaceutical composition **characterized in that** such pharmaceutical composition comprises a synergetic combination of an anti-microbial agent, such as: Ciprofloxacin and an antioxidant agent, known as: Ascorbic Acid (Vitamin C), as well as pharmaceutically acceptable excipients; wherein the active principles are present in the formulation in a concentration range from 250.0 mg to 1.0 gr for Ciprofloxacin, and from 50.0 mg to 200.0 mg for Ascorbic Acid; which are formulated in a single dosage unit for oral administration, indicated for the treatment of urinary infections.

2. The pharmaceutical composition of claim 1, **characterized in that** the anti-microbial agent, such as the active principle: Ciprofloxacin is present in the formulation in a concentration range from 250.0 mg to 1.0 gr, preferably, a concentration of 500.0 mg per dosage unit is used in the formulation.

3. The pharmaceutical composition of claims 1 and 2, **characterized in that** the antioxidant agent, such as the active principle: Ascorbic Acid (Vitamin C) is present in the formulation in a concentration range from 50.0 mg to 200.0 mg, preferably, a concentration of 100.0 mg per dosage unit is used in the formulation.

4. The pharmaceutical composition of claims 1 to 3, **characterized in that** such composition is formulated in a single dosage unit intended for oral administration in the form of capsules and tablets.

5. The pharmaceutical composition of claims 1 to 4, **characterized in that** such composition is for use in the treatment of Urinary Infections.

## Patentansprüche

1. Pharmazeutische Zusammensetzung, **dadurch gekennzeichnet, dass** solche pharmazeutische Zusammensetzung eine synergetische Kombination aus einem antimikrobiellen Mittel, wie: Ciprofloxacin und einem Antioxidationsmittel, bekannt als: Ascorbinsäure (Vitamin C), sowie pharmazeutisch akzeptable Hilfsstoffe umfasst; wobei die Wirkstoffe in der Formulierung in einem Konzentrationsbereich von 250,0 mg bis 1,0 g für das Ciprofloxacin, und von 50,0 mg bis 200,0 mg für die Ascorbinsäure enthalten sind; welche in einer einzelnen Dosierungseinheit zur oralen Verabreichung formuliert sind, die für die Behandlung von Harnwegsinfektionen geeignet ist.

2. Pharmazeutische Zusammensetzung nach Anspruch 1, **dadurch gekennzeichnet, dass** das antimikrobielle Mittel, wie der Wirkstoff Ciprofloxacin, in der Formulierung in einem Konzentrationsbereich von 250,0 mg bis 1,0 g enthalten ist, vorzugsweise, eine Konzentration von 500,0 mg pro Dosierungseinheit in der Formulierung verwendet wird.

3. Pharmazeutische Zusammensetzung nach den Ansprüchen 1 und 2, **dadurch gekennzeichnet, dass** das Antioxidationsmittel, wie der Wirkstoff Ascorbinsäure (Vitamin C) in der Formulierung in einem Konzentrationsbereich von 50,0 mg bis 200,0 mg enthalten ist, vorzugsweise, eine Konzentration von 100,0 mg pro Dosierungseinheit in der Formulierung verwendet wird.

4. Pharmazeutische Zusammensetzung nach den Ansprüchen 1 bis 3, **dadurch gekennzeichnet, dass** solche Zusammensetzung in einer einzelnen Dosierungseinheit formuliert ist, die zur oralen Verabreichung in Form von Kapseln und Tabletten bestimmt ist.

5. Verwendung der pharmazeutischen Zusammensetzung nach den Ansprüchen 1 bis 4, **dadurch gekennzeichnet, dass** solche Zusammensetzung für deren Verwendung bei der Behandlung von Harnwegsinfektionen ist.

## Revendications

1. Composition pharmaceutique **caractérisée en ce qu'**une telle composition pharmaceutique comprend une combinaison synergétique d'un agent antimicrobien, tel que : ciprofloxacine et un agent antioxydant, bien connu comme: acide ascorbique (Vitamine C), ainsi que des excipients pharmaceutiquement acceptables ; dans laquelle les principes actifs sont présents dans la formulation dans un intervalle de concentration allant de 250,0 mg à 1,0 g pour la ciprofloxacine, et de 50,0 mg à 200,0 mg pour l'acide ascorbique ; lesquels sont formulés dans une seul unité de dosage pour administration orale, indiquée pour le traitement d'infections urinaires.

2. Composition pharmaceutique selon la revendication 1, **caractérisé en ce que** l'agent antimicrobien, en tan que principe actif : la ciprofloxacine est présente dans la formulation dans un intervalle de concentration allant de 250,0 mg a 1,0 g, de préférence, une concentration de 500,0 g par unité de dosage est utilisée dans la formulation.

3. Composition pharmaceutique selon les revendications 1 et 2, **caractérisée en ce que** l'agent antioxydant, en tant que principe actif : l'acide ascorbique (vitamine C) est présent dans la formulation dans un intervalle de concentration allant de 50,0 mg a 200,0 mg, de préférence, une concentration de 100,0 mg par unité de dosage est utilisée dans la formulation.

4. Composition pharmaceutique selon les revendications 1 à 3, **caractérisée en ce qu'**une telle composition est formulée en une seule unité de dosage conçue pour administration orale sous la forme de capsules et de comprimés.

5. Composition pharmaceutique selon les revendications 1 à 4, **caractérisée en ce qu'**une telle composition est destinée à être employée dans le traitement d'infections urinaires.
